(19) Europäisches Patentamt
European Patent Office
Office européen des brevets

(11) **EP 1 752 487 A1**

(12) **EUROPEAN PATENT APPLICATION**
published in accordance with Art. 158(3) EPC

(43) Date of publication:
**14.02.2007 Bulletin 2007/07**

(21) Application number: **05745929.9**

(22) Date of filing: **25.05.2005**

(51) Int Cl.:
***C08J 9/26*** (2006.01)    ***A61N 1/30*** (2006.01)
***C08L 101/00*** (2006.01)

(86) International application number:
**PCT/JP2005/010010**

(87) International publication number:
**WO 2005/116123 (08.12.2005 Gazette 2005/49)**

(84) Designated Contracting States:
**DE GB**

(30) Priority: **27.05.2004 JP 2004157683**

(71) Applicant: **TOKUYAMA CORPORATION**
**Shunan-shi, Yamaguchi-ken 745-8648 (JP)**

(72) Inventors:
• **FUKUTA, Kenji, Tokuyama Corporation**
**Shunan-shi, Yamaguchi 745-8648 (JP)**

• **SAKATA, Kanji, Tokuyama Corporation**
**Shunan-shi, Yamaguchi 745-8648 (JP)**

(74) Representative: **Cresswell, Thomas Anthony**
**J.A. KEMP & CO.**
**14 South Square**
**Gray's Inn**
**London WC1R 5JJ (GB)**

(54) **PROCESS FOR PRODUCING ION EXCHANGE MEMBRANE FOR IONTOPHORESIS**

(57) A method of producing an ion-exchange membrane for iontophoresis by preparing a polymerizable composition containing monomer components and non-polymerizable components which are compatible with the monomer components but do not copolymerize therewith; forming the polymerizable composition into a membrane form; polymerizing the polymerizable composition in the obtained membrane form to thereby form a membrane of a structure in which the non-polymerizable components are dispersed in the polymer having a crosslinked structure; removing the non-polymerizable components from the obtained membrane; and, as required, introducing ion-exchange groups into the polymer. The ion-exchange membrane obtained by the above method is applied to an iontophoresis device. When an ionic medicine contained in a medicine-containing portion of the device is permeated into a living body by the electrophoresis through the ion-exchange membrane, not only ionic medicines having small formula weights but also medicinal ions of formula weights of about 500 to about 1,000 can be efficiently administered into the living body.

EP 1 752 487 A1

**Description**

Field of the Invention

The present invention relates to a method of producing an ion-exchange membrane used in the iontophoresis for permeating, into the living body, an ionic medicine useful for the living body by utilizing the electrophoresis.

Background Art

The iontophoresis for permeating, into the living body, an ionic medicine useful for the living body by utilizing the electrophoresis has been widely known as a method of administering a medicine of a required amount into a diseased part in a pain-free state.

In the iontophoresis, so far, a medicine-containing layer imbibing an ionic medicine is placed on the living body, a working electrode is arranged on the side opposite to the living body with the medicine layer sandwiched therebetween, a counterelectrode is placed on the living body separated away from the medicine-containing layer, and an electric current is permitted to flow across the working electrode and the counter electrode from a power source causing the ionic medicine to permeate into the living body. This method has an object of permeating the ionic medicine only into the living body through the living body interface such as the skin and the mucous membrane. According to this method, however, the ionic medicine does not necessarily pass through the living body interface, but, conversely, it often happens that sodium cations, potassium cations and chloride anions permeate back into the medicine layer from the side of the living body. In particular, ionic medicines that are considered to be useful for the living body have a smaller mobility than those of ions existing in the living body, and a desired medicine is not efficiently administered (does not efficiently permeate into the living body) in proportion to the amount the electric current is supplied. In the iontophoresis, further, the medicine comes into direct contact with the electrodes triggering a reaction on the electrodes not only wasting the medicine but also forming compounds that may adversely affect the living body. Moreover, the medicine is usually imbibed in the form of an aqueous solution. Therefore, the electrolysis of water takes place on the working electrode and on the counter electrode, whereby the pH of the medicine-containing aqueous solution varies due to $H^+$ ions and $OH^-$ ions that are formed often causing the living body to be inflamed.

In order to solve these problems, new iontophoretic methods have been proposed by arranging an ion-exchange membrane on the living body interface so that medicinal ions permeate into the living body through the ion-exchange membrane (e.g., see patent documents 1 to 4).

[Patent document 1] JP-A-3-94771
[Patent document 2] JP-T-3-504343
[Patent document 3] JP-A-4-297277
[Patent document 4] JP-A-2000-229128

According to the systems proposed in the above patent documents, the ion-exchange membrane arranged on the living body interface permits the permeation of only those ions having the same polarity as the desired medicinal ions. This makes it possible to prevent the ions having a polarity opposite to that of medicinal ions of the desired medicine from oozing out of the living body and, hence, to accomplish a high dosage of the medicine as compared with when no ion-exchange membrane is arranged. The above technologies use a commercial ion-exchange membrane which employs, as a reinforcing member (base member), a woven fabric, that is used for the manufacture of the salt and for the dialysis of food compounds.

According to the study conducted by the present inventors, however, when the ion-exchange membrane having the above woven fabric as a base member is used, the medicine can be administered in an increased amount as compared with when the ion-exchange membrane is not used, but the amount of administration is still insufficient. Particularly, ionic medicines having relatively small ionic formula weights, such as ascorbic acid (salt) and histamine (salt) can be administered relatively favorably. It was, however, learned that the administering efficiency sharply decreases as the medicinal ions of the ionic medicine increase, and becomes very poor as the ionic formula weight exceeds about 500.

Summary of the Invention

It is, therefore, an object of the present invention to provide a method of producing an ion-exchange membrane for iontophoresis which is capable of efficiently administering, into the living body, not only ionic medicines having small formula weights but also ionic medicines of which the medicinal ions have large formula weights.

In an attempt to solve the above problems, the present inventors have conducted the study extensively. As a result, the inventors have discovered the fact that an ion-exchange membrane capable of efficiently administering medicinal ions of an ionic medicine having a large ionic formula weight can be obtained when the ion-exchange membrane is produced by polymerizing a polymerizable monomer composition containing a non-polymerizable component and, thereafter, removing the non-polymerizable component, and have finished the present invention.

According to the present invention, there is provided a method of producing an ion-exchange membrane for iontophoresis comprising the steps of:

(I) preparing a polymerizable composition containing monomer components (a) inclusive of a polyfunctional polymerizable monomer having a plurality of polymerizable functional groups, and non-polymerizable components (b) which are compatible with the monomer components (a) but do not copolymerize therewith;
(II) forming the polymerizable composition into a membrane form;
(III) polymerizing the polymerizable composition in the membrane form obtained in the step (II) above to thereby form a membrane of a structure in which the non-polymerizable components are dispersed in a polymer having a crosslinked structure;
(IV) removing the non-polymerizable components from the membrane obtained in the step (III) above; and
(V) introducing ion-exchange groups into the polymer having the crosslinked structure when the monomer components do not include the monomer that has the ion-exchange groups.

According to the present invention, there is further provided an iontophoresis device comprising (A) a working electrode structure having a working electrode, a medicine-containing portion and an ion-exchange membrane obtained by the above production method, (B) an counter electrode structure having a counter electrode opposing the working electrode, and (C) a power source unit electrically connected to the working electrode structure and to the counter electrode structure, wherein an ionic medicine contained in the medicine-containing portion permeates into the living body by the electrophoresis through the ion-exchange membrane.

The ion-exchange membrane obtained by the above production method of the invention is capable of efficiently administering, into the living body, not only ionic medicines having small formula weights but also medicinal ions of formula weights of about 500 to about 1, 000. The iontophoresis device using the above ion-exchange membrane does not almost permit ions to permeate back to the side of the medicine layer from the side of the living body. Therefore, the device works very excellently for percutaneously administering medicinal ions of relatively high molecular weights.

Brief Description of the Drawings

Fig. 1 is a view schematically illustrating a representative structure of an iontophoresis device using an ion-exchange membrane produced by the present invention; and
Fig. 2 is a view schematically illustrating a device used for measuring the amounts of administering the medicine according to the embodiment.

Best Mode for Carrying Out the Invention

If briefly described, in the present invention the ion-exchange membrance for iontophoresis is produced by preparing a polymerizable composition containing a non-polymerizable component (step (I)), forming the polymerizable composition into a membrane form (step (II)); polymerizing the polymerizable composition in the obtained membrane form to thereby form a membrane of a structure in which the non-polymerizable component is dispersed in the polymer having a crosslinked structure (step (III)); removing the non-polymerizable component from the obtained membrane (step (IV)); and, as required, introducing ion-exchange groups into the polymer (step (V)). When the ion-exchange membrane produced by the above method is applied to an iontophoresis device and the ionic medicine contained in the medicine-containing portion of the device is permeated into the living body by the electrophoresis through the ion-exchange membrane, not only ionic medicines having small formula weights but also medicinal ions of formula weights of about 500 to about 1,000 can be efficiently administered into the living body. That is, since the non-polymerizable component is removed, the ion-exchange membrane as a whole becomes relatively porous. As a result, it is considered that not only ionic medicines having small formula weights but also ionic medicines having large formula weights easily permeate through the membrane, and are efficiently administered into the living body. With the ion-exchange membrane obtained by a conventional method, for example, the membrane as a whole becomes dense. Therefore, though the ionic medicines having small formula weights are allowed to pass through the membrane, the ionic medicines having large formula weights cannot easily pass through the membrane, and efficient administration is not accomplished.

[Step (I) of preparing a polymerizable composition]

The invention, first, prepares a polymerizable composition comprising monomer components (a) and non-polymerizable components (b) which do not copolymerize with the monomer components.

Monomer components (a):

The monomer components (a) are for forming an ion-exchange resin which is a main component of the ion-exchange membrane. As the monomer components (a), it is necessary to use a polyfunctional polymerizable monomer (crosslinking monomer) having two or more polymerizable functional groups for forming a crosslinked structure. It is, further allowable to use the crosslinking monomer alone as the monomer component (a). Usually, however, a monofunctional polymerizable monomer is used in combination with the crosslinking monomer to easily form a membrane and to obtain an ion-exchange membrane having a suitable degree of flexibility.

The monofunctional polymerizable monomer has one polymerizable functional group, and there is used the one having a functional group into which an ion-exchange group can be introduced or the one having an ion-exchange group.

As the monofunctional polymerizable monomer having a functional group into which an ion-exchange group can be introduced, there can be used, without any particular limitation, a hydrocarbon-type monomer that has heretofore been used for the production of ion-exchange resins. As the hydrocarbon-type monomers having a functional group into which a cation-exchange group can be introduced, there can be exemplified aromatic vinyl compounds such as styrene, $\alpha$-methylstyrene, 3-methylstyrene, 4-methylstyrene, 2,4-dimethylstyrene, p-tert-butylstyrene, $\alpha$-halogenated styrene and vinylnaphthalene. As the hydrocarbon-type monomers having a functional group into which an anion-exchange group can be introduced, there can be exemplified styrene, vinyltoluene, chloromethylstyrene, vinylpyridine, vinylimidazole, $\alpha$-methylstyrene and vinylnaphthalene. These monofunctional polymerizable monomers can be used in a single kind or in two or more kinds in combination.

In the monofunctional polymerizable monomer having an ion-exchange group, further, there is no particular limitation on the ion-exchange group provided it is a functional group capable of possessing a negative or positive electric charge in an aqueous solution. As the cation-exchange group, there can be generally exemplified sulfonic acid group, carboxylic acid group and phosphonic acid group and, particularly preferably, the sulfonic acid group which is a strongly acidic group. As the anion-exchange group, further, there can be exemplified primary to tertiary amino groups, quaternary ammonium group, pyridyl group, imidazole group, quaternary pyridinium group, and quaternary imidazolium group and, particularly preferably, quaternary ammonium group and quaternary pyridinium group which are strongly basic groups.

As the monofunctional polymerizable monomer having the cation-exchange group, there can be exemplified sulfonic acid-type monomers such as styrenesulfonic acid, vinylsulfonic acid and $\alpha$-halogenated vinylsulfonic acid; carboxylic acid-type monomers such as methacrylic acid, acrylic acid and maleic anhydride; phosphonic acid-type monomers such as vinylphosphoric acid, as well as salts thereof and esters thereof. As the monofunctional polymerizable monomer having the anion-exchange group, further, there can be exemplified amine-type monomers such as vinylbenzyltrimethylamine, and vinylbenzyltriethylamine; nitrogen-containing heterocyclic monomers such as vinylpyridine and vinylimidazole, as well as salts thereof and esters thereof. The monofunctional polymers having the ion-exchange groups may be used in a single kind or in two or more kinds in combination.

As the crosslinking monomer, further, there can be used polyfunctional polymerizable monomers without any particular limitation provided they are capable of copolymerizable with the above monofunctional polymerizable monomers. For example, there can be used polyfunctional vinyl compounds such as divinylbenzenes, divinylsulfone, butadiene, chloroprene, divinylbiphenyl, and trivinylbenzene; and polyfunctional methacrylic acid derivatives such as trimethylolmethane trimethacrylic acid ester, methylenebisacrylamide and hexamethylenedimethacrylamide.

In the present invention, there is no particular limitation on the ratio of the above polymerizable monomers used as the monomer component (a). Generally, however, the crosslinking monomer is used in an amount of 0.1 to 50 parts by mass and, preferably, 1 to 40 parts by mass per 100 parts by mass of the monofunctional polymerizable monomer having an ion-exchange group or a functional group into which the ion-exchange group can be introduced. It is also allowable to use any other monomer copolymerizable with the above monomers, such as acrylonitrile, acrolein or methyl vinyl ketone in an amount of not larger than 100 parts by weight.

The present invention uses a monomer having a functional group into which an ion-exchange group can be introduced. When the monomer having the ion-exchange group is not used, it becomes necessary to introduce the ion-exchange group into the obtained polymer in the step (V) that will be described later.

Non-polymerizable components (b):

The present invention uses the non-polymerizable components (b) together with the above monomer components (a). The non-polymerizable components (b) are those that to be removed from the polymer formed by polymerizing the monomer components (a) and, therefore, must not copolymerize with the monomer components (a) though they are compatible with the monomer components (a). That is, if the non-polymerizable components (b) are not compatible with the monomer components (a), porous portions are nonuniformly formed in the ion-exchange membrane that is finally obtained and the permeation of ions through the membrane becomes unstable. Further, if the non-polymerizable components (a) copolymerize with the monomer components (b), then, the non-polymerizable components (b) cannot be removed from the polymer. In the present invention, the non-polymerizable components (b) compatible with the monomer components (a) stand for that when the two are mixed together, there exists a ratio of amounts at which the two form a homogeneous solution without being separated into two phases. Therefore, a perfectly homogeneous solution does not have to be formed at all mixing ratios.

There is no particular limitation on the non-polymerizable components (b) provided they satisfy the above-mentioned conditions, and there can be exemplified various organic solvents and polymers (hereinafter referred to as non-copolymerizable polymers) that do not copolymerize with the above monomers.

As the organic solvent, there can be exemplified alcohols such as methanol, ethanol, 1-butanol, 2-ethoxyethanol; aliphatic hydrocarbons such as hexane, cyclohexane, heptane and 1-octane; fatty acids such as octanoic acid; amines such as dimethylactylamine; aromatic hydrocarbons such as toluene, xylene and naphthalene; ketones such as acetone, cyclohexanone and methyl ethyl ketone; ethers such as dibenzyl ether and diethylene glycol dimethyl ether; halogenated hydrocarbons such as methylene chloride, chloroform and ethylene bromide; alcohol esters of aromatic acids and aliphatic acids, such as dimethyl phthalate, dioctyl phthalate, dimethyl isophthalate, dibutyl adipate, triethyl citrate, acetyltributyl citrate and dibutyl sebacate; and alkylphosphoric esters, which may be used in a single kind or in two or more kinds in combination. By taking the compatibility with the monomer components (a) and the polymerization temperature (not to volatilize at the polymerization temperature) into consideration, the organic solvent can be suitably selected depending upon the kinds and compositions of the monomer components (a). Particularly preferably, however, there can be used 1-butanol, 2-ethoxyethanol, dibenzyl ether, dioctyl phthalate and acetyltributyl citrate.

As the non-copolymerizable polynomer, further, there can be exemplified styrene/butadiene copolymer, acrylonitrile/butadiene copolymer, polybutadiene and polypropylene glycol. They, too, are suitably selected by taking the compatibility with the monomer components (a) into consideration and depending upon the kinds and compositions of the monomer components (a). Particularly preferably, however, there are used polybutadiene and polypropylene glycol. Further, the non-copolymerizable polymer has an average molecular weight of, generally, 500 to 10, 000 and, preferably, 500 to 6,000. Though it may vary depending upon its amount of addition, when the non-copolymerizable polymer has an average molecular weight of smaller than 500, however, the medicinal ions having large formula weights are less administered. When the non-copolymerizable polymer has an average molecular weight that exceeds 10,000, on the other hand, the effect decreases for repelling ions in the living body having a sign opposite to that of the medicinal ions, which is not desirable, either.

As the non-polymerizable components (b), either the above organic solvent or the non-copolymerizable polynomer may be used. Generally, however, it is desired to use both of them being mixed together. When the organic solvent only is used, the medicinal ions having large formula weights cannot be sufficiently administered unless the amount of addition is increased. Therefore, the effect is insufficient for repelling ions of the opposite sign. When the non-copolymerizable polynomer only is used, further, it becomes difficult to completely remove the non-copolymerizable polynomer from the polymer of the monomer components (a), and the medicinal ions having large formula weights are not often administered to a sufficient degree.

For example, it is desired that the non-copolymerizable component (b) are used in an amount of 5 to 200 parts by mass and, particularly, 10 to 150 parts by mass per 100 parts by mass of the monomer component (a). It is, further, desired that the organic solvent and the non-copolymerizable monomer are used in combination at a mass ratio of 100: 20 to 100:500 and, particularly, 100: 50 to 100: 300. When the amount of the organic solvent or of the non-copolymerizable polymer is smaller than the above range, the medicinal ions having large formula weights cannot be administered to a sufficient degree. When the amount thereof is greater than the above range, on the other hand, the effect for repelling ions of the opposite sign tends to decrease.

In the present invention, the polymerizable composition can be easily prepared by mixing the monomer components (a) and the non-polymerizable components (b) together. Generally, however, the polymerizable composition is blended with a polymerization initiator to efficiently proceed the polymerization. As the polymerization initiator, though there is no particular limitation, there is usually used an organic peroxide such as octanoyl peroxide, lauroyl peroxide, t-butylperoxy-2-ethyl hexanoate, benzoyl peroxide, t-butylperoxyisobutylate, t-butylperoxylaurate, t-hexylperoxybenzoate or di-t-butyl peroxide. The polymerization initiator is blended in an amount of 0.1 to 20 parts by mass and, particularly, 0.5 to 10 parts by mass per 100 parts by mass of the monofunctional polymerizable monomer in the monomer components (a). As required, further, the polymerizable composition may be blended with known additives such as plasticizer and the like.

[Step (II) of forming a membrane]

In the present invention, next, the polymerizable composition is formed into a membrane form. The membrane form can be formed by any known method. From the standpoint of easy production and excellent mechanical strength of the ion-exchange membrane that is finally obtained, however, it is desired to form the membrane form by bringing the polymerizable composition into contact with the porous base member in the form of a sheet or a film, and permeating polymerizable composition into the voids in the porous base member. The porous base member works to impart the shape to the membrane. Besides, use of the above porous base member makes it very easy to accomplish both a high strength for preventing breakage during the storage and use and flexibility for favorably following up the shape of skin at the time of use.

There is no particular limitation on the porous base member. Usually, a paper, a woven fabric, a nonwoven fabric or a porous film is used. From the standpoint of obtaining the ion-exchange membrane having a small thickness and a high mechanical strength (from the standpoint of efficiently administering the medicine while effectively preventing the breakage), in particular, it is desired to use a nonwoven fabric or a porous film as the porous base member and it is most desired to use the porous film as the porous base member.

The porous film has many fine pores penetrating through from the front surface to the back surface, and is, usually, made of a thermoplastic resin. As the thermoplastic resin, there can be used polyolefin resins such as homopolymers or copolymers of $\alpha$-olefins like ethylene, propylene, 1-butene, 1-pentene, 1-hexene, 3-methyl-1-butene, 4-methyl-1-pentene, and 5-methyl-1-heptene; vinyl chloride resins such as polyvinyl chloride, vinyl chloride/vinyl acetate copolymer, vinyl chloride/vinylidene chloride copolymer, and vinyl chloride/olefin copolymer; fluorine-contained resins such as polytetrafluoroethylene, polychlorotrifluoroethylene, vinylidene polyfluoride, tetrafluoroethylene/hexafluoropropylene copolymer, tetrafluoroethylene/perfluoroalkylvinyl ether copolymer and tetrafluoroethylene/ethylene copolymer; polyamides such as nylon 6 and nylon 66; and polyimide resin. It is, however, desired to use a polyolefin resin, particularly, polyethylene or polypropylene and, most desirably, polyethylene from the standpoint of mechanical strength, flexibility, chemical stability and resistance against the chemicals.

There is no particular limitation on the property of the porous film comprising the above thermoplastic resin. From the standpoint of obtaining an ion-exchange membrane having a small electric resistance and a large physical strength, however, it is desired that the pores have an average diameter of, preferably, 0.005 to 5.0 $\mu$m, more preferably, 0.01 to 2.0 $\mu$m and, most preferably, 0.02 to 0.2 $\mu$m. The above average porous diameter stands for a value measured in compliance with the Bubble Point Method. Similarly, it is desired that the percentage of voids of the porous film is, preferably, 20 to 95% and, more preferably, 30 to 90% and, most preferably, 30 to 60%. Further, the physical strength of the obtained ion-exchange membrane increases with an increase in the thickness of the porous film. When the thickness becomes too large, however, the electric resistance of the obtained ion-exchange membrane increases while follow-up property to the surface of the living body decreases. It is, therefore, desired that the thickness of the porous film is in a range of 5 to 150 $\mu$m and, particularly, 10 to 120 $\mu$m.

The porous film of the above thermoplastic resin can be obtained according to the methods taught in JP-A-9-212964 and JP-A-2002-338721. Concretely speaking, the porous film is prepared by mixing an organic liquid to a thermoplastic resin to form it into a sheet or a film and, then, extracting the organic liquid therefrom by using a solvent, or by drawing a sheet of a thermoplastic resin filled with an inorganic filler and/or an organic filler. The porous drawn film is further available in the market (for example, "Hipore" manufactured by Asahi Kasei Co., "U-Pore" manufactured by Ube Kosan Co., "Setela" manufactured by Tonen Tapils Co., "Expole" manufactured by Mitsubishi Kagaku Co., "Hilet" manufactured by Mitsui Kagaku Co., etc.).

As the nonwoven fabric used as the porous base member, there can be used those produced by a dry method and a wet method without any particular limitation. As the material of the nonwoven fabric, there can be used, for example, polyester fiber, polypropylene fiber, polyamide fiber, nylon fiber, acrylic fiber, rayon fiber, vinylon fiber or polyurethane fiber. Though there is no particular limitation on the properties of the nonwoven fabric, it is desired that the nonwoven fabric has a weight of 20 to 100 g/m$^2$ and an apparent thickness of 30 to 250 $\mu$m from the standpoint of obtaining an ion-exchange membrane having a small thickness, excellent strength and low electric resistance.

There is no particular limitation on the method of permeating the polymerizable composition into the porous base member. Usually, however, an application method, a spray method or an immersion method is employed. When the polymerizable composition is to be applied or sprayed, however, a method may be employed to bring the two into contact under a reduced pressure or to apply pressure after the contact, so that the voids in the porous base member are favorably filled with the solution.

[Step of polymerization (III)]

In the present invention, the polymerizable composition in the membrane form obtained through the step (II) is polymerized. As polymerization means, a method is preferably employed according to which the membrane form of the polymerizable composition is polymerized by elevating the temperature from normal temperature while pressing the membrane form by holding it with films having smooth surfaces, such as polyester films. Upon conducting the polymerization while holding the membrane form with films, the polymerization is prevented from being adversely affected by oxygen in the environment, and the surfaces after the polymerization can be smoothed. The polymerizing conditions may be suitably determined depending upon the kind of the polymerization initiator that is used and the composition of the monomer. Usually, a state of being heated at about 80 to about 120°C is maintained for about 5 minutes to about 10 hours. The polymerization is conducted under the conditions where the non-polymerizable components (b) that are used do not volatilize or are not decomposed.

In conducting the polymerization, the non-polymerizable components (b) contained in the polymerizable composition do not copolymerize with the monomer components (a) but are compatible with the monomer components (a). Therefore, a membrane (hereinafter simply called cured membrane) is formed having a structure in which a polymer having a crosslinked structure serves as a matrix and the non-polymerizable components (b) are just homogeneously dispersed in the polymer.

[Step (IV) of removing the non-polymerizable components (b)]

In the present invention, the non-polymerizable components (b) are removed from the cured membrane obtained above, and a desired ion-exchange membrane is obtained. That is, upon removing the non-polymerizable components (b) dispersed in the cured product, the obtained ion-exchange membrane becomes porous as a whole enabling even those medicinal ions having large formula weights to migrate therein relatively easily.

There is no particular limitation on the method of removing the non-polymerizable components (b); i.e., the non-polymerizable components (b) may be suitably removed depending upon the kind of the components (b) that are used and the amounts thereof. For example, when a non-polymerizable polymer or a nonvolatile (sparingly volatile) organic solvent is used (or when these two are used in combination) as the components (b), the non-polymerizable components (b) are eluted by being immersed in an easily volatile solvent capable of dissolving them and, thereafter, the easily volatile solvent is volatilized to remove the components (b).

When the non-polymerizable components (b) are to be removed by being dissolved in a solvent, there can be used any solvent without limitation provided it exhibits wettability to the cured membrane and is capable of dissolving the non-polymerizable components (b). Concretely speaking, there can be exemplified highly volatile organic solvents among those exemplified as organic solvents that can be used as the components (b). They are suitably selected depending upon the wettability to the cured membrane and the solubility of the non-polymerizable components (b). Concretely, there can be preferably used methanol, ethanol, acetone, methyl ethyl ketone, toluene, xylene, methylene chloride and chloroform.

[Step of introducing ion-exchange groups (V)]

In the present invention, when the monomer having the ion-exchange group is not used as the monomer components (a), the above-mentioned ion-exchange group must be introduced into the polymer formed by using the monomer components (a).

The treatment for introducing the ion-exchange groups may be conducted by suitably selecting a known method. Typically, a method of introducing the ion-exchange groups may be followed, which is, usually, employed in the method of producing hydrocarbon-type ion-exchange membranes. For example, when the cation-exchange membrane is to be obtained, a treatment may be conducted such as sulfonation, chlorosulfonation, phosphoniumation or hydrolysis. When the anion-exchange membrane is to be obtained, on the other hand, a treatment may be conducted such as amination or alkylation.

The ion-exchange groups can be introduced before or after the step (IV) of removing the non-copolymerizable components (b), or in parallel with the step (IV) of removal. When the non-copolymerizable polymer or the nonvolatile (or sparingly volatile) organic solvent is used as the non-copolymerizable components (b), or when the non-polymerizable components (b) are lowly miscible with a reagent that is used for introducing the ion-exchange groups, it is desired to conduct the treatment for introducing the ion-exchange groups after the step (IV) in order to highly efficiently introduce the ion-exchange groups. When the non-polymerizable components (b) are used, however, the components (b) may be substituted for the easily volatile solvent or for the solvent miscible with the reagent that is used for introducing the ion-exchange groups.

The ion-exchange membrane obtained by the above production method has a structure in which, for example, the porous base member is filled with the porous ion-exchange resin, has a feature in that medicinal ions having large ionic formula weights are allowed to pass through easily, and can be effectively applied to an iontophoresis device.

In the ion-exchange membrane, the filling ratio of the ion-exchange resin in the membrane may vary depending upon the percentage of voids of the porous base member that is used or the amount of the components (b) that are blended but is, usually, in a range of 5 to 95% by mass and is, preferably, in a range of 10 to 90% by mass in order to facilitate the permeation of medicinal ions and to increase the strength of the ion-exchange membrane.

It is, further, desired that the ion-exchange membrane contains the ion-exchange groups in an amount of 0.1 to 6.0 mmols/g, and, particularly, 0.3 to 4.0 mmols/g as the ion-exchange capacity from the standpoint of lowering the electric resistance of the ion-exchange membrane to lower a voltage that is needed for administering the ionic medicine.

It is further desired that the ion-exchange membrane has a water content of not smaller than 5% and, preferably, not smaller than 10% so that its electric resistance will not increase due to drying. Usually, the water content is maintained to be about 5 to about 90%. The water content in the above range can be obtained by being controlled relying upon a known method such as relying upon the kind of the ion-exchange groups, upon the ion-exchange capacity and upon the degree of crosslinking. In order to highly efficiently administer the desired medicine, further, it is desired that the ion-exchange membrane has a fixed ion concentration of 3.0 to 15.0 mmols/g - water.

It is further desired that, upon using the porous base member having the thickness as described above, the ion-exchange membrane has a thickness of, generally, 5 to 150 $\mu$m and, particularly, 10 to 130 $\mu$m so as to exhibit well-balanced properties with regard to physical strength of the membrane, electric resistance of the membrane and following up the surface of the living body.

[Iontophoresis Device]

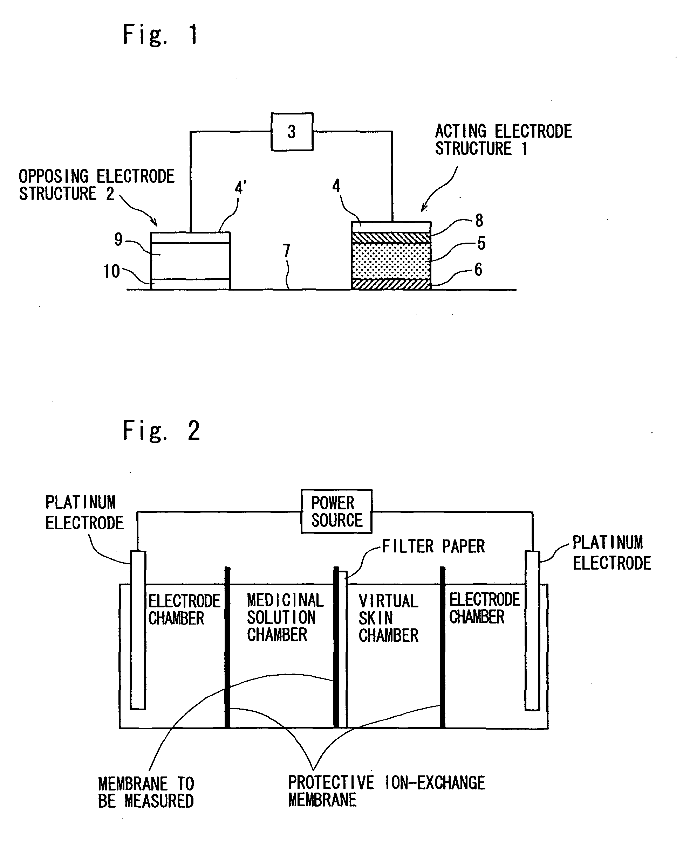

The ion-exchange membrane obtained by the above-mentioned production method can be applied to an intophoresis device that is used for administering the ionic medicine into the living body by utilizing the electrophoresis including a step of permitting the ionic medicine to pass through the ion-exchange membrane. Fig. 1 illustrates a representative structure of the iontophoresis device.

As shown in Fig. 1, the iontophoresis device is constituted by a working electrode structure 1, a counter electrode structure 2, and a power source unit 3 electrically connected to these structures. The ion-exchange membrane obtained by the above-mentioned production method can be favorably used for the working electrode structure 1.

(Working electrode structure 1)

The working electrode structure 1 includes an electrode (working electrode) 4 that serves as a working electrode, a medicine-containing portion 5 containing an ionic medicine, and an ion-exchange membrane 6 produced by the above-mentioned method. The ion-exchange membrane 6 selectively permits the permeation of ions of the same polarity as the medicinal ions of the ionic medicine to be administered. In the working electrode structure 1 as shown in Fig. 1, there are arranged the working electrode 4, medicine-containing portion 5 and ion-exchange membrane 6 in this order. Usually, these members are laminated in an armoring material (not shown) to constitute the working electrode structure 1, and the ion-exchange membrane 6 is arranged to be positioned on a living body interface (skin) 7.

An ion-exchange membrane 8 may further be included between the electrode and the medicine-containing layer to prevent the decomposition of the medicine to be administered and to prevent the pH of the medicine-containing portion 5 from being varied by the electrode reaction. It is desired that the ion-exchange membrane 8 is the one which selectively permits the passage of ions of a polarity opposite to that of the medicinal ions.

As required, further, an ion-permeating sheet of an ionically conducting gel, a porous film or a woven fabric may be provided between the ion-exchange membrane 6 and the living body interface 7. The gel or the sheet may assume a structure integral with the working electrode structure 1. Or, the gel or the sheet may be held relative to the living body interface 7 only at the time of use. Though not illustrated, the working electrode structure 1 may further include an ionically conducting gel, an electrolytic solution, or a porous film or a woven fabric imbibing the electrolytic solution between the working electrode 4 and the ion-exchange membrane 8.

As the working electrode 4 in the working electrode structure 1, there can be used, without limitation, any electrode that is usually used in the electrochemical processes. For example, there can be used an electrode comprising an electronically conducting material such as gold, platinum, silver, copper, nickel, zinc or carbon, or a self-sacrificing electrode such as semiconductor electrode or silver/silver chloride, which may be used alone or in combination. Preferably, there can be exemplified gold, platinum, silver and carbon. These electrodes may be plates, sheets, meshes or an amorphous laminate fibers, which is shaped and worked like a paper, or may be the one obtained by plating or vaporizing an electrode member on an ion-exchange membrane.

As the medicine-containing portion 5 in the working electrode structure 1, there can be used, without any limitation, a medicine-containing layer that is used in the ordinary iontophoresis. That is, there can be used a solution obtained by dissolving an ionic medicine in a solvent such as water or ethanol, a gel obtained by mixing the above solution with a polyvinyl alcohol or a polyvinyl pyrrolidone, or the one of a porous film or a gauze imbibing the above solution. There is no particular limitation on the ionic medicine contained in the medicine-containing portion 5. The ionic medicine may be any substance that comprises cations and anions and exhibits medicinal effect as the positive ions or negative ions enter into the living body.

Examples of the ionic medicine of which the positive ions exhibit the effect include anesthetics such as procaine hydrochloride, lidocaine hydrochloride and dibucaine hydrochloride; anti-malignant tumor agents such as mitomycin and pleomycin hydrochloride; anodynes such as morphine hydrochloride; steroids such as medroxyprogesterone acetate; histamine and insulin. As the ionic medicine of which the negative ions exhibit the effect, there can be exemplified vitamin agents such as vitamin B2, vitamin B12, vitamin C, vitamin E and folic acid; anti-inflammatory agents such as aspirin and ibuprofen; adrenocortical hormones such as dexamethasone-type water-soluble compounds; and antibiotics such as benzylpenicillin potassium.

The ion-exchange membrane 6 is obtained by the above-mentioned production method. Use of the above ion-exchange membrane 6 makes it possible to efficiently administer even medicinal ions having a large ionic formula weight of, for example, 300 to 1,500 and, particularly, 400 to 1, 000 into the living body. When, for example, an ion-exchange membrane produced by a conventional method is used, it is not allowed to efficiently administer the medicinal ions having a large ionic formula weight since the membrane is densely formed.

When the above iontophoresis device is used in such a manner that the ion-exchange membrane 6 comes into contact with the living body surface such as the skin, it is desired that the ion-exchange membrane 6 is so smooth as to favorably maintain an intimate contact.

(Counter electrode structure 2)

The counter electrode structure 2 has an electrode (counter electrode) 4' that opposes the working electrode 4 of the working electrode structure 1 and can assume, without any limitation, a structure used for a portion including an electrode that becomes a counter electrode in an ordinary iontophoresis device. That is, the counter electrode structure 2 may be the electrode (counter electrode 4') itself, may be a structure in which the electrode (counter electrode 4') is arranged on a sheet of an ionically conducting gel, a porous film or a woven fabric, or may be a structure in which the electrode (counter electrode 4') is arranged on an ion-exchange membrane using a porous film as the base member or on any other ion-exchange membrane. Preferably as shown in Fig. 1, the counter electrode 4', an electrolyte-containing portion 9 containing an electrolyte and an ion-exchange membrane 10 are laminated in this order, the ion-exchange membrane 10 being arranged on the living body interface. In this case, the ion-exchange membrane 10 may be an ion-exchange membrane formed by the above-mentioned method or by any other method. It is, however, desired that the ion-exchange membrane 10 is an ion-exchange membrane formed by using a porous film as the porous base member from the standpoint of its excellent mechanical strength. Further, the ion-exchange membrane 10 may be the one which selectively permits the permeation of ions of a polarity same as, or opposite to, that of the medicinal ions of the desired medicine. Preferably, however, the ion-exchange membrane 10 is the one that selectively permeates ions of the polarity opposite to that of the medicinal ions of the desired medicine to prevent the permeation of the desired medicine into the counter electrode structure from the living body.

The electrolyte-containing portion 9 in the counter electrode structure 2 may be a solution itself obtained by dissolving an electrolyte in a solvent such as water or an ethanol, a gel obtained by mixing the above solution with a polyvinyl alcohol or a polyvinyl pyrrolidone, or the one of a porous film or a gauze imbibing the above solution. There can be used any ionic electrolyte without limitation, such as sodium chloride or potassium chloride, if it dissolves in a solvent such as water or ethanol and exhibits ionic property.

Further, like in the case of the working electrode structure 1, the ocounter electrode structure 2 may be provided with an ion-exchange membrane between the counter electrode 4' and the ion-exchange membrane 10, may be provided with a sheet capable of permeating ions comprising an ionically conducting gel, a porous film or a woven fabric between the ion-exchange membrane 10 and the living body interface, or may be provided with an ionically conducting gel or an electrolytic solution or with a porous film or a woven fabric imbibing the electrolytic solution between the counter electrode 4' and the ion-exchange membrane closest thereto.

(Power source unit 3)

As the power source unit 3, there can be used any power source unit that is used in an ordinary iontophoresis device without limitation. When the working electrode structure 1, counter electrode structure 2 and the power source unit 3 are independent from each other, there can be used an external power source that can be connected to a battery or to a power source of the system. In such a case, it is desired to use in combination a power source control system such as a system for stabilizing the voltage or the current or a system for applying a pulse current.

When the iontophoresis device of the present invention is to be realized in a portable form, it is desired to use a cell as the power source. As the cell, there can be exemplified a coin type silver oxide cell, an air-zinc cell or a lithium ion cell. By using the above small cell as a power source, there can be realized an iontophoresis device which is small in size and easy to carry incorporating the working electrode structure 1, the counter electrode structure 2 and the power source unit 3 in an armoring material.

EXAMPLES

The invention will be described more concretely by way of the following Examples and Comparative Examples to which only, however, the invention is in no way limited. Properties of the ion-exchange membranes shown in Examples and Comparative Examples were measured by the methods described below.

(1) Ion-exchange capacity, water content and fixed ion concentration:

The ion-exchange membrane was immersed in a 1 (mol/l) HCl aqueous solution for not less than 10 hours.
Thereafter, in the case of the cation-exchange membrane, the hydrogen ion type was substituted by the sodium ion type with a 1 (mol/l) NaCl aqueous solution, and the liberated hydrogen ions were determined with a sodium hydroxide aqueous solution by using a potential-difference titration device (COMTITE-900, manufactured by Hiranuma Sangyo Co.)(A mols). In the case of the anion-exchange membrane, on the other hand, a chloride ion type was substituted by a nitric acid ion type with a 1 (mol/1) NaNO$_3$ aqueous solution, and the liberated chloride ions were determined with a silver nitrate aqueous solution by using the potential-difference titration device (COMTITE-900, manufactured by Hiranuma Sangyo Co.)(A mols).
Next, the same ion-exchange membrane was immersed in a 1 (mol/l) HCl aqueous solution for not less than 4 hours, and was washed with ion-exchanged water to a sufficient degree. The membrane was taken out, water on the surfaces thereof was wiped with a tissue paper or the like, and the weight (W g) thereof when wet was measured. Next, the membrane was dried at 60°C for 5 hours under a reduced pressure, and the weight was measured (D g). Based on the above measured values, the ion-exchange capacity, water content and fixed ion concentration were found in compliance with the following formulas,

$$\text{Ion-exchange capacity} = A \times 1000/D \ [\text{mmol/g} - \text{dry weight}]$$

$$\text{Water content} = 100 \times (W - D)/D \ [\%]$$

$$\text{Fixed ion concentration} = \text{ion-exchange capacity/water content} \times 100 \ [\text{mmol/g} - \text{water}]$$

(2) Membrane resistance.

An ion-exchange membrane was held in a two-chamber cell equipped with a platinum black electrode, a 0.5 (mol/l) sodium chloride aqueous solution was filled on both sides of the ion-exchange membrane, a resistance across the electrodes was measured relying on an AC bridge (frequency of 1000 cycles/sec) at 25°C, and the membrane resistance was found relying upon a difference between the resistance across the electrodes and the resistance across the electrodes of when no ion-exchange membrane was set up. The membrane used for the measurement had been equilibrated in advance in a 0.5 (mol/l) sodium chloride aqueous solution.

(3) Surface roughness.

The surface roughness on the surface of the ion-exchange membrane was measured by using a three-dimensional roughness measuring instrument (Model TDF-3A, manufactured by Kosaka Kenkyujo Co.). On a diagram of the obtained roughness distribution, a difference in height was measured from the deepest portion neighboring the peak portion, and an average value of differences in the height measured over a length of 11 mm was regarded to be the surface roughness of the ion-exchange membrane.

(4) Amount of permeation of medicine.

An aqueous solution containing 10% by weight of a polyvinyl alcohol (NH-20 manufactured by Nihon Gosei Co.) was applied onto a Teflon sheet in such a manner that the thickness of the polyvinyl alcohol film after the solvent was removed was 6 μm. Thereafter, water was removed by drying conducted at 150°C for 10 minutes to obtain a virtual skin. Next, a filtering paper (filtering paper 5C for chemical analysis manufactured by Advantech Co.), the virtual skin, the ion-exchange membrane to be measured and protective ion-exchange membranes that prevent the medicine from arriving at the electrode, were set in a cell shown in Fig. 2, and the medicine solution chamber was filled with an aqueous solution of medicine of a predetermined concentration, a virtual skin chamber was filled with an aqueous solution of 0.9% by mass of sodium chloride, and the two electrode chambers were filled with a 0.1 (mol/l) sodium lactate aqueous solution. As the protective ion-exchange membrane, there was used an anion-exchange membrane obtained in Comparative Example 1 when the ion-exchange membrane to be measured was the cation-exchange membrane, and there was used a cation-exchange membrane obtained in Comparative Example 2 when the object to be measured was the anion-exchange membrane.

Next, an electric current was supplied for 3 hours at a predetermined constant current density or at a constant voltage while maintaining the cell at 25°C and stirring the medicine solution chamber and the virtual skin chamber. After the end of supplying the electric current, the solution in the virtual skin chamber was readily drained and the amount of medicine was measured relying on a liquid chromatography. The same operation was executed without supplying the electric current to measure a blank value. A difference from the amount of medicine of when the current was supplied was calculated, and was regarded to be the amount the medicine that has permeated.

(Preparation Example 1)

A monomer mixture was prepared by using the following polymerizable monomers (monomer components (a)) according to the following recipe.

Monomer mixture:

| | |
|---|---|
| Chloromethylstyrene (polymerizable monomer): | 380 g |
| Divinylbenzene (polymerizable monomer): | 20 g |
| t-Butylperoxyethyl hexanoate (polymerization initiator): | 20 g |

200 Grams of a dibenzyl ether which is an organic solvent was added as a non-polymerizable component (b) to the above monomer mixture to thereby prepare a polymerizable composition.

620 Grams of the polymerizable composition was introduced into a 1000-ml glass container and in which a porous film (polyethylene of a weight average molecular weight of 250,000, film thickness of 25 $\mu$m, an average porous size of 0.03 $\mu$m, percentage of voids of 37%) measuring 20 cm x 20 cm was immersed therein under the atmospheric pressure at 25°C for 10 minutes, and the polymerizable composition was imbibed by the porous film.

Next, the porous film was taken out from the polymerizable composition, and both sides of the porous film were covered with a polyester film of 100 $\mu$m, followed by the thermal polymerization under a nitrogen pressure of 3 kg/cm$^2$ at 80 °C for 5 hours to obtain a membrane. Next, the obtained membrane was immersed in methanol for 24 hours and the dibenzyl ether was extracted and removed, followed by the reaction in an aminating bath comprising 10 parts by weight of trimethylamine of 30% by weight, 5 parts by weight of water and 5 parts by weight of acetone at room temperature for 5 hours to obtain a quaternary ammonium-type anion-exchange membrane.

The obtained anion-exchange membrane was measured for its ion-exchange capacity, water content, fixed ion concentration, membrane resistance, membrane thickness and surface roughness. The results were as shown in Table 1.

(Preparation Examples 2 to 5, Comparative Preparation Example 1)

Anion-exchange membranes were prepared in the same manner as in Preparation Example 1 but changing the polymerizable monomers (components (a)), non-polymerizable component (b) and porous film into those of compositions shown in Table 1. Properties of the obtained membranes were as shown in Table 1.

(Preparation Examples 6, 7 and Comparative Preparation Example 2)

Porous films were filled with monomer-mixed compositions shown in Table 1 in the same manner as in Preparation Example 1. Next, the porous films were taken out from the mixtures, and both sides of the porous films were covered with a polyester film of 100 $\mu$m, followed by the thermal polymerization under a nitrogen pressure of 3 kg/cm$^2$ at 80 °C for 5 hours. The obtained membranes were immersed in acetone for 24 hours and were dried. Thereafter, the membranes were immersed in a mixture of sulfuric acid of 98% and chlorosulfonic acid of a purity of not lower than 90% at a ratio of 1:1 at 40°C for 45 minutes to obtain sulfonic acid-type cation-exchange membranes.

The obtained cation-exchange membranes were measured for their ion-exchange capacities, water contents, fixed ion concentrations, membrane resistances, membrane thickness and surface roughness. The results were as shown in Table 1.

Table 1

| | Porous films | Composition (weight ratio) | | | | | |
| | | Components (a) | | | Components (b) | | |
| Comp. Prep. Ex. | | CMS | St | DVB | DBE | PPG | PO |
|---|---|---|---|---|---|---|---|
| 1 | A | 95 | 0 | 5 | 50 | 0 | 5 |
| 2 | A | 95 | 0 | 5 | 0 | 70 | 5 |
| 3 | A | 95 | 0 | 5 | 30 | 70 | 5 |
| 4 | A | 97.5 | 0 | 2.5 | 50 | 70 | 5 |
| 5 | B | 95 | 0 | 5 | 5 | 70 | 5 |
| 6 | A | 0 | 80 | 20 | 50 | 70 | 5 |
| 7 | A | 95 | 90 | 10 | 30 | 70 | 5 |
| Comp. Prep. Ex. 1 | A | 0 | 0 | 5 | 0 | 0 | 5 |
| Comp. Prep. Ex. 2 | A | 0 | 90 | 10 | 0 | 0 | 5 |
| Neosepta AMX | woven fabric | | | | | | |
| Neosepta CMX | woven fabric | | | | | | |

Table 1 (continued)

| Comp. Prep. Ex. | Properties of ion-exchange membranes | | | | | | | |
|---|---|---|---|---|---|---|---|---|
| | Ion-exchange group | Ion-exchange capacity [mmol/g - dry membrane] | Water content [%] | Fixed ion concentration [mmol/g - water] | Membrane resistance [$\Omega \cdot cm^2$] | Membrane thickness [μm] | Surface roughness [μm] | Bursting strength [MPa] |
| 1 | quaternary ammonium type | 1.4 | 24 | 5.8 | 0.6 | 28 | 1 or less | 0.40 |
| 2 | quaternary ammonium type | 1.2 | 22 | 5.5 | 0.8 | 26 | 1 or less | 0.40 |
| 3 | quaternary ammonium type | 1.1 | 24 | 4.6 | 0.7 | 26 | 1 or less | 0.38 |
| 4 | quaternary ammonium type | 1.0 | 24 | 4.2 | 0.7 | 26 | 1 or less | 0.38 |
| 5 | quaternary ammonium type | 1.4 | 40 | 3.5 | 0.4 | 29 | 1 | 0.20 |
| 6 | sulfonic acid type | 1.4 | 27 | 5.2 | 0.8 | 26 | 1 or less | 0.39 |
| 7 | sulfonic acid type | 1.6 | 28 | 5.7 | 0.5 | 29 | 1 or less | 0.38 |
| Comp. Prep. Ex. 1 | quaternary ammonium type | 1.8 | 22 | 8.2 | 0.5 | 32 | 1 or less | 0.37 |
| Comp. Prep. Ex. 2 | sulfonic acid type | 2.4 | 29 | 8.3 | 0.5 | 31 | 1 or less | 0.38 |
| Neosepta AMX | quaternary ammonium type | 1.5 | 25 | 6.0 | 2.5 | 150 | 11 | 0.42 |
| Neosepta CMX | sulfonic acid type | 1.6 | 28 | 5.7 | 2.2 | 160 | 12 | 0.45 |

\<note\>

● Porous films:

A: Polyethylene having a weight average molecular weight of 250,000, a film thickness of 25 $\mu$m, an average pore size of 0.03 $\mu$m and a percentage of voids of 37%.

B: Polypropylene having a weight average molecular weight of 200,000, a film thickness of 27 $\mu$m, an average pore size of 0.2 $\mu$m and a percentage of voids of 50%.

● CMS: Chloromethylstyrene.

● 4-VP: 4-Vinylpyridine.

● St: Styrene.

● DVB: Divinylbenzene

● DBE: Dibenzyl ether

● PPG: Polypropylene glycol (diol type, average molecular weight, 3,000)

● PO: t-Butylperoxyethyl hexanoate.

(Examples 1 to 5)

The amounts of permeation of the medicine were measured by using a virtual skin under the conditions of using ion-exchange membranes (membranes to be measured) prepared in Preparation Examples 1 to 5, filling a medicinal solution chamber of the testing apparatus of Fig. 2 with a 10 mmol/l solution of an ascorbic acid phosphate magnesium salt, and flowing a current of a density of 0.5 mA/cm$^2$ constant. The results were as shown in Table 2.

(Comparative Example 1)

The amount of permeation of the medicine was measured in the same manner as in Example 1 but using the Neosepta AMX (manufactured by Tokuyama Corp., membrane properties are as described in Table 1) which was an anion-exchange membrane as the ion-exchange membrane having, as the base member, the woven fabric used in the conventional iontophoresis. The results were as shown in Table 2.

(Comparative Example 2)

The amount of permeation of the medicine was measured in the same manner as in Example 1 but using the anion-exchange membrane of Comparative Preparation Example 1 that was prepared without using the non-polymerizable components (b). The results were as shown in Table 2.

(Comparative Example 3)

The amount of permeation of the medicine was measured in the same manner as in Example 1 by using the virtual skin only but without using the ion-exchange membrane to be measured. The results were as shown in Table 2.

Table 2

|  | Ion-exchange membranes to be measured | Medicine concentration [mmol/l] | Current density [mA/cm$^2$] | Amount of permeation [$\mu$mol/cm$^2$] |
|---|---|---|---|---|
| Example 1 | membrane of Prep. Ex. 1 | 10 | 0.5 | 10.0 |
| Example 2 | membrane of Prep. Ex. 2 | 10 | 0.5 | 13.4 |
| Example 3 | membrane of Prep. Ex. 3 | 10 | 0.5 | 12.2 |
| Example 4 | membrane of Prep. Ex. 4 | 10 | 0.5 | 11.8 |
| Example 5 | membrane of Prep. Ex. 5 | 10 | 0.5 | 7.5 |
| Comparative Example 1 | Neosepta AMX | 10 | 0.5 | 2.5 |
| Comparative Example 2 | membrane of Comp. Prep. Ex. 1 | 10 | 0.5 | 5.8 |
| Comparative Example 3 | none | 10 | 0.5 | 0.5 |
| Medicine: Ascorbic acid phosphate magnesium salt | | | | |

(Examples 6 to 9)

Amounts of permeation of the medicine were measured in the same manner as in Example 1 by using ion-exchang membranes shown in Table 3 and a 10 mmol/l solution of a dexamethasone phosphate sodium salt. The results were as shown in Table 3.

(Comparative Example 4)

The amount of permeation of the medicine was measured in the same manner as in Example 6 but using the Neosepta AMX (manufactured by Tokuyama Corp., membrane properties are as described in Table 1) which was an anion-exchange membrane as the ion-exchange membrane having, as the base member, the woven fabric used in the conventional iontophoresis. The results were as shown in Table 3.

(Comparative Example 5)

The amount of permeation of the medicine was measured in the same manner as in Example 6 but using the anion-exchange membrane of Comparative Preparation Example 1 that was prepared without adding the non-polymerizable components (b). The results were as shown in Table 3.

(Comparative Example 6)

The amount of permeation of the medicine was measured in the same manner as in Example 6 by using the virtual skin only but without using the ion-exchange membrane to be measured. The results were as shown in Table 3.

Table 3

|  | Ion-exchange membranes to be measured | Medicine concentration [mmol/l] | Current density [mA/cm$^2$] | Amount of permeation [$\mu$mol/cm$^2$] |
|---|---|---|---|---|
| Example 6 | membrane of Prep. Ex. 2 | 10 | 0.5 | 1.8 |

(continued)

|  | Ion-exchange membranes to be measured | Medicine concentration [mmol/l] | Current density [mA/cm$^2$] | Amount of permeation [$\mu$mol/cm$^2$] |
|---|---|---|---|---|
| Example 7 | membrane of Prep. Ex. 3 | 10 | 0.5 | 2.3 |
| Example 8 | membrane of Prep. Ex. 4 | 10 | 0.5 | 2.0 |
| Example 9 | membrane of Prep. Ex. 5 | 10 | 0.5 | 1.9 |
| Comparative Example 4 | Neosepta AMX Neosepta AMX | 10 | 0.5 | 0 |
| Comparative Example 5 | membrane of Comp. Prep. Ex. 2 | 10 | 0.5 | 0.02 |
| Comparative Example 6 | none | 10 | 0.5 | 1.5 |
| Medicine: Dexamethasone phosphate sodium salt | | | | |

(Examples 10, 11, Comparative Examples 7, 8)

Amounts of permeation of the medicine were measured by using the ion-exchange membranes shown in Table 4 and a 10 mmol/l solution of a lidocaine hydrochloride which is a cationic medicine. The results were as shown in Table 4.

Table 4

|  | Ion-exchange membranes to be measured | Medicine concentration [mmol/l] | Current density [mA/cm$^2$] | Amount of permeation [$\mu$mol/cm$^2$] |
|---|---|---|---|---|
| Example 10 | membrane of Prep. Ex. 6 | 10 | 0.5 | 19 |
| Example 11 | membrane of Prep. Ex. 7 | 10 | 0.5 | 21 |
| Comparative Example 7 | Neosepta CMX | 10 | 0.5 | 17 |
| Comparative Example 8 | none | 10 | 0.5 | 1.3 |
| Medicine: Lidocaine hydrochloride | | | | |

**Claims**

1.    A method of producing an ion-exchange membrane for iontophoresis comprising the steps of:

(I) preparing a polymerizable composition containing monomer components (a) inclusive of a polyfunctional polymerizable monomer having a plurality of polymerizable functional groups, and non-polymerizable components (b) which are compatible with said monomer components (a) but do not copolymerize therewith;
(II) forming said polymerizable composition into a membrane form;
(III) polymerizing the polymerizable composition in the membrane form obtained in said step (II) to thereby form a membrane of a structure in which said non-polymerizable components (b) are dispersed in the polymer having a crosslinked structure;
(IV) removing said non-polymerizable components from the membrane obtained in said step (III); and
(V) introducing ion-exchange groups into the polymer having said crosslinked structure when said monomer components do not include the monomer that has the ion-exchange groups.

2. A method of producing an ion-exchange membrane for iontophoresis according to claim 1, wherein said monomer components include a monofunctional polymerizable monomer and said polyfunctional polymerizable monomer.

3. A method of producing an ion-exchange membrane for iontophoresis according to claim 2, wherein, as said monofunctional polymerizable monomer, use is made of a monomer having an ion-exchange group or without having an ion-exchange group but is capable of introducing the ion-exchange group.

4. A method of producing an ion-exchange membrane for iontophoresis according to claim 1, wherein in said step (II), said polymerizable composition is formed into a membrane form by causing said polymerizable composition to permeate into voids in a sheet-like or film-like porous base member.

5. A method of producing an ion-exchange membrane for iontophoresis according to claim 1, wherein an organic solvent or a non-copolymerizable polymer is used as said non-polymerizable components (b).

6. A method of producing an ion-exchange membrane for iontophoresis according to claim 5, wherein at least any one of those selected from the group consisting of 1-butanol, 2-ethoxyethanol, dibenzyl ether, dioctyl phthalate and acetyltributyl citrate is used as said organic solvent.

7. A method of producing an ion-exchange membrane for iontophoresis according to claim 5, wherein at least any one of those selected from the group consisting of styrene/butadiene copolymer, acrylonitrile/butadiene copolymer, polybutadiene and polypropylene glycol is used as said non-copolymerizable polymer.

8. A method of producing an ion-exchange membrane for iontophoresis according to claim 1, wherein in said step (IV), said non-copolymerizable components (b) are eluted in volatile solvent, and said volatile solvent is volatilized to remove said non-copolymerizable components.

9. An iontophoresis device comprising:

(A) a working electrode structure having a working electrode, a medicine-containing portion and an ion-exchange membrane obtained by the production method of claim 1;
(B) a counter electrode structure having a counter electrode opposing said working electrode; and
(C) a power source unit electrically connected to the working electrode structure and to the counter electrode structure;
wherein an ionic medicine contained in said medicine-containing portion permeates into the living body by the electrophoresis through said ion-exchange membrane.

Fig. 1

ACTING ELECTRODE
STRUCTURE 1

OPPOSING ELECTRODE
STRUCTURE 2

Fig. 2

PLATINUM
ELECTRODE

POWER
SOURCE

FILTER PAPER

PLATINUM
ELECTRODE

ELECTRODE
CHAMBER

MEDICINAL
SOLUTION
CHAMBER

VIRTUAL
SKIN
CHAMBER

ELECTRODE
CHAMBER

MEMBRANE TO
BE MEASURED

PROTECTIVE ION-EXCHANGE
MEMBRANE

## INTERNATIONAL SEARCH REPORT

| International application No. |
| --- |
| PCT/JP2005/010010 |

A. CLASSIFICATION OF SUBJECT MATTER
Int.Cl⁷ C08J9/26, A61N1/30//C08L101:00

According to International Patent Classification (IPC) or to both national classification and IPC

B. FIELDS SEARCHED

Minimum documentation searched (classification system followed by classification symbols)
Int.Cl⁷ C08J9/26, A61N1/30

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 10-158416 A (Tokuyama Corp.), 16 June, 1998 (16.06.98), Claims; Par. Nos. [0010] to [0011] (Family: none) | 1-8 |
| A | JP 2-251230 A (Tokuyama Soda Co., Ltd.), 09 October, 1990 (09.10.90), Claims; page 2, lower right column, line 7 to page 3, lower left column, line 9, lower right column, line 5 to page 4, upper right column, line 10 (Family: none) | 1-8 |

| ☒ Further documents are listed in the continuation of Box C. | ☐ See patent family annex. |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 11 August, 2005 (11.08.05) | 30 August, 2005 (30.08.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

| | INTERNATIONAL SEARCH REPORT | International application No. |
|---|---|---|
| | | PCT/JP2005/010010 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 53-82867 A  (Mitsubishi Chemical Industries Ltd.), 21 July, 1978 (21.07.78), Claims; page 2, upper left column, line 10 to upper right column, line 20; page 3, lower left column, lines 11 to 18 | 1-8 |
| A | JP 51-128390 A  (Asahi Chemical Industry Co., Ltd.), 09 November, 1976 (09.11.76), Claims; page 2, upper right column, line 10 to page 3, upper left column, line 20, lower right column, line 3 to page 4, upper right column, line 16; page 6, lower right column, line 13 to page 7, upper left column, line 5 & US 4093570 A          & US 4154917 A | 1-8 |
| A | JP 2000-229128 A  (R and R Ventures Kabushiki Kaisha), 22 August, 2000 (22.08.00), Claims (Family: none) | 9 |
| P,A | JP 2004-188188 A  (Tokuyama Corp.), 08 July, 2004 (08.07.04), Claims; Par. Nos. [0041] to [0054] & WO 2004/047916 A1 | 9 |

Form PCT/ISA/210 (continuation of second sheet) (January 2004)

| INTERNATIONAL SEARCH REPORT | International application No. |
| --- | --- |
| | PCT/JP2005/010010 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:

because they relate to subject matter not required to be searched by this Authority, namely:

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

A special technical feature of the inventions described in claims 1 to 8 relates to a process for producing an ion exchange membrane for iontophoresis, while a special technical feature of the invention described in claim 9 relates to an iontophoresis apparatus.

The process according to the inventions described in claims 1 to 8 is not a process particularly suitable for the production of the apparatus according to the invention described in claim 9.

Accordingly, claims 1 to 8 and claim 9 are not in a technical relationship involving one or more of the same or corresponding special technical
(continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**          ☐  The additional search fees were accompanied by the applicant's protest.

☒  No protest accompanied the payment of additional search fees.

Form PCT/ISA/210 (continuation of first sheet (2)) (January 2004)

**INTERNATIONAL SEARCH REPORT**

International application No.

PCT/JP2005/010010

Continuation of Box No.III of continuation of first sheet(2)

features and thus are not so linked as to form a single general inventive concept.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3094771 A
- JP 3504343 T
- JP 4297277 A
- JP 2000229128 A
- JP 9212964 A
- JP 2002338721 A